# EUROPEAN PATENT APPLICATION

(11) **EP 4 467 128 A1**
(43) Date of publication of application: **27.11.2024**
(21) Application number: 24176688.0
(22) Date of filing: 17.05.2024
(51) Int. Cl.: A61K 9/10, A61K 47/12

(54) **METHOD FOR PREPARING AN INTRAMAMARY TEAT SEALING COMPOSITION AND SEALING COMPOSITION OBTAINED BY SUCH PROCESS**

(30) Priority: 18.05.2023 BR 102023009678
(71) Applicant: Filid Tecnologia Farmaceutica Ltda., 06454-020 Barueri SP (BR)
(72) Inventor: MASCARO, Claudio, 18190-000 ARAÇOIABA DA SERRA (BR)
(74) Representative: Studio Torta S.p.A.

(57) **Abstract**

The present invention is about a method of preparation and a pharmaceutical form of an injectable intramammary teat sealing composition for use in the veterinary industry.

Intramammary teat sealant composition, which comprises organic glyceryl monostearate or calcium stearate, with particle size between 30 and 100µm, in addition to mineral oil, titanium dioxide and stabilizer, in the absence of water.

## Description

### FIELD OF APPLICATION

This invention deals with a method of preparation and pharmaceutical form of an injectable intramammary teat sealant composition for use in the veterinary industry.

### STATE OF THE ART

Mastitis is an infection of the udder caused by one of several bacterial pathogens (streptococci, staphylococci, coliforms) entering the mammary gland through the teat canal when the sphincter is relaxed after milking or suckling.

This is the most prevalent infectious disease in adult dairy cattle. Mastitis causes great economic damage and loss to dairy industries around the world.

The dairy cow's mammary gland requires a period of non-lactation when it approaches calving, in order to optimize milk production in the subsequent lactation. This period is called the "dry period". Generally, 40 to 60 days are recommended. After the dry period, there is a transition from the non-lactating state to the lactating state, called the pre-calving period. The lactation period that follows the pre-calving period lasts approximately 300 - 310 days (Alany, R.G., Bhattarai, S., Pranatharthiharan, S., Devarajan, P.V. (2013). Intramammary Delivery Technologies for Cattle Mastitis Treatment. In: Rathbone, M., McDowell, A. (eds) Long Acting Animal Health Drug Products. Advances in Delivery Science and Technology. Springer, New York, NY).

Preventing new infections during the drying period before the cessation of lactation is a major challenge for dairy producers. The non-lactating udder is prone to bacterial infection, with rates of new infections being highest at the beginning of the dry period and approaching calving. After drying, the closure of the teat canal by the formation of a keratin plug greatly influences the incidence of intramammary infections (IMI) during the dry period. At present, antibiotic therapy at the end of lactation is the most effective means of eliminating existing infections and preventing new ones.

For this reason, mastitis is the most frequent cause for antibiotic use on dairy farms and contributes to total drug and veterinary costs.

Despite the long history of using antibiotics to treat IMI in cows, however, preventing new infections appears to offer greater long-term benefits. The success rate of using antibiotics is very low and there are a number of problems associated with their use in treating IMI. A limitation of the present therapeutic approach for the dry period is that it provides little or no protective effect against new infections during the pre-calving period. Most available dry cow products do not persist until the end of the dry period and consequently the udder is susceptible to further IMI during the pre-calving period. Efforts to control mastitis through management practices have not been shown to reduce new infections in the dry period. Furthermore, the presence of antibiotic residues in milk for human consumption is a major concern in today's dairy industry. Furthermore, the multiple uses of antibiotics for therapeutic or prophylactic purposes in the dry period may lead to the emergence of antibiotic resistance. Antibiotic use in animals is often cited as a potential cause of antibiotic resistance in humans, since antibiotic-resistant bacteria can be transferred to humans through animal feed.

Recently, non-antibiotic prophylactic agents that act as physical barriers to seal the teat internally during the drying period have been developed as an alternative method of controlling IMI.

Conventional internal teat seals have shown some promising results in preventing new cases of mastitis during the dry period when infused into teats.

Document US6254881 originally describes the preparation of a pharmaceutical formulation containing bismuth nitrate (65% bismuth subnitrate) and aluminum monostearate in a liquid paraffin base and infused from a plastic syringe, similar in type to those used for intramammary antibiotic infusion. Studies have shown that this bismuth-based intramammary teat is as effective as dry cow antibiotic therapy for preventing new infections in the dry period.

Although the clinical use of bismuth subnitrate is approved by the EMEA (Committee for Products of Veterinary Medicine), however, following the introduction of the "ORBESEAL" brand internal teat sealant product in the USA, visual imperfections in aged dairy products, most notably aged cheddar cheeses are starting to appear. Visual imperfection takes the form of small black spots (approximately 0.5 to 5 mm in diameter) that appear throughout the aged cheese.

Spots are purely aesthetic visual imperfections that reduce the quality (and consequently the market value) of the cheese affected by the problem. The spots are not accompanied by any organoleptic imperfections in the cheese. Affected cheese with black spots can be sold, although to a lesser extent than unaffected cheeses. The imperfection was called "black spot imperfections" in document BRPI0719273A2.

The veterinary formulation developed and described in document BRPI0923252 comprises the use of vegetable oils associated with bismuth subnitrate, which can be paste or gel, with paraffin, aluminum stearate and silicon dioxide in the composition.

In another sense, document BRPI1016082 seeks to explore gel formation technology containing a glyceride base, magnesium stearate and a metallic salt such as titanium dioxide, zinc oxide or barium sulfate. This invention is based on the non-use of bismuth subnitrate.

There are also publications on internal teat sealants prepared with methyl cellulose, cetyl alcohol, liquid paraffin, glycerin, methyl paraben and gentian violet (Elecko J, Federic F and Vrtiak OJ (1985) Dry period teat seal in the prevention of intramammary infections. Proceedings of 5th International symposium on mastitis control. pp. 225-234).

Another formulation combines bismuth subnitrate with chlorhexidine - a powerful microbicide (Compton CWR, Emslie FR and Mcdougall S (2014) Randomized controlled trials demonstrating efficacy of a novel internal teat sealant to prevent new intramammary infections in dairy cows and heifers. New Zealand Veterinary Journal 62, 258-266); (Petrovski KR, Caicedo-Caldas A, Williamson NB, Lopez-Villalobos N, Grinberg A, Parkinson TJ and Tucker IG (2011) Efficacy of a novel internal dry period teat sealant containing 0.5% chlorhexidine against experimental challenge with Streptococcus uberis in dairy cattle. Journal of Dairy Science 94, 3366-3375).

Another development of internal teat sealants belongs to New AgriTech Enterprises, NY, through its commercial product Cinnatube^{R}, composed of herbal oil, beeswax, olive oil, tea tree oil, calendula, cinnamon and eucalyptus (Mullen KAE, Anderson KL and Washburn SP (2014) Effect of 2 herbal intramammary products on milk quantity and quality compared with conventional and no dry cow therapy. Journal of Dairy Science 97, 3509-3522). There is also an association with essential oils.

In addition to this limitation, the price and availability of bismuth subnitrate make the formulation more expensive than many commercially available intramammary administration methods, the products are poorly formulated, suffering from problems of physical stability and poor syringability.

For these reasons there is a need to develop a more appropriate pharmaceutical form for better prevention of mastitis and a subsequent reduction in the use of antibiotics.

### PURPOSES OF THE INVENTION

In this sense, the objective was to develop a simple and applicable method for preparing and using a physical intramammary barrier. The method involves infusing a sealant formulation into the cow's teat.

The formulation developed according to the present invention is free from bismuth subnitrate and does not cause black spot defects in dairy products generated from animal milk. The sealant does not contain antibiotics or active anti-infective agents.

### UNUSUAL EFFECT

The sealing composition according to the invention showed good stability, good syringability, in addition to being effective in combating mastitis.

### BRIEF DESCRIPTION

The present invention describes a method of preparing a sealing composition and intramammary teat sealing composition comprised of organic salt of glyceryl monostearate or calcium stearate, with particle size between 30 and 100 µm, preferably between 30 and 60 µm, in addition to mineral oil, anatase titanium dioxide and stabilizer, in the absence of water.

The method of preparing the sealing composition comprises the following steps:
- micronizing glyceryl monostearate or salt derived from stearic acid, calcium, to a particle size between 30 and 100 µm (component a);
- dissolving the preservatives in the mineral oil while stirring;
- slowly adding, while stirring, the micronized calcium stearate (or component a); and then slowly adding anatase titanium dioxide under stirring;
- maintaining constant stirring between 80 and 120 rpm for 1 to 3 h.
- unloading the product and filling it into syringes.

### DETAILED DESCRIPTION

The innovative method according to the present invention may comprise the use of organic salt of glyceryl monostearate or salt derived from non-toxic calcium stearic acid. More specifically, the organic salt is calcium stearate.

The appropriate base may comprise the use of mineral oil and titanium dioxide.

The composition according to the present invention may be comprised of said organic salt, by mass, between 20 to 70% of the total mass of the sealing composition.

According to one form of attaining the invention, the sealing composition comprises in mass in relation to the total mass of the composition:
- mineral oil between 30 and 70.0% (m/m);
- calcium stearate between 20 and 50.0% (m/m);
- titanium dioxide between 2.0 to 10.0% (m/m);
- preservatives between 0.05 to 0.60% (m/m).

The preservatives contained in the formulation can be chosen from the classes of anisoles and parabens.

According to another form or attaining of the invention, the sealing composition may comprise:
- mineral oil between 30 and 70.0% (m/m);
- calcium stearate between 20 and 50.0% (m/m);
- titanium dioxide between 2.0 to 10.0% (m/m);
- butyl hydroxy toluene 0.05 to 0.30% (m/m);
- butylhydroxyanisole 0.05 to 0.30% (m/m).

With a specific objective, the prepared formulation is intended for the prevention of mastitis, being an internal sealant for cow teats.

This invention provides a new veterinary product based on the association of a salt derived from a fatty acid, namely, calcium stearate, and a base, which can be a mineral oil, to combat mastitis in cows. This product is a pasty, intramammary injectable and stable pharmaceutical formulation.

Another important aspect of the invention is related to the particle size of calcium stearate, which has a direct influence on viscosity and syringability. According to the present invention, in order to obtain the desired result, the particle size of the calcium stearate must be between 30 and 100 µm, more preferably between 30 and 60 µm.

The process of the present invention consists of preparing a base system at room temperature; dissolving calcium stearate therein and then adding titanium dioxide, stirring at 50 to 200 RPM.

Thus, in this inventive process, a pharmaceutically stable formulation is obtained.

The present invention, therefore, presents an easy and simple way to prepare the formulation, which, as it is a paste, allows it to be filled into appropriate syringes, avoiding precipitation of the product in the form of undesirable granules that could interfere with the average dosage injected into the animal. This formulation also, being stable, provides excellent syringability, resulting in shorter handling time, absence of syringe clogging and less stress for the animals to be treated.

It is known that some formulations for internal sealing of teats, over time, harden and form calcified products inside the teat, ending up making it unusable and in many cases, difficult to clean and releasing residues into the milk, such as specifically from bismuth subnitrate which presents black spots in dairy products derived from this milk.

The present invention overcomes this obstacle.

Based on these chemical and pharmacological concepts, the present invention solves the problem of incompatibility, chemical and residual degradation of excipients through their correct choice and association, in addition to providing a pharmaceutical form with safe and effective prolonged action. The formulation is physically and chemically stable, does not contain water in its composition, with a pH compatible with intramammary tissue.

According to another embodiment of the present invention, the intramammary sealing composition comprises:
- mineral oil between 40 and 60.0% (m/m);
- calcium stearate between 40 and 50.0% (m/m);
- titanium dioxide between 2.0 to 4.0% (m/m);
- butyl hydroxy toluene 0.05 to 0.30% (m/m);
- butylhydroxyanisole 0.05 to 0.30% (m/m).

The preferred components and their concentration range are illustrated in Table 1 below:

**TABLE 1**

| Ingredient | Role | Percentage (m/m) (%) |
|---|---|---|
| Mineral oil | Wetting base excipient | 40.0 to 60.0 |
| Calcium stearate | Water repellent | 40.0 to 50.0 |
| Titanium dioxide | Pigment | 2.0 to 4.0% |
| Butyl hydroxy toluene and Butylhydroxyanisole | Preservative | 0.05 to 0.30 |

Another aspect that concerns this invention is the acceptability of the viscosity of the formulation for use in syringes. Following the preparation of the pharmaceutical formulation, it presents excellent syringability, which is the ability of a product to be successfully handled through an appropriate syringe or needle. Syringability tests were conducted successfully.

The formulations were exhaustively tested, and during the studies, an appropriate formulation was found with excellent stability, ideal viscosity, conditions that are commercially desired.

Pharmaceutical Form Preparation Method

According to the present invention, the method of preparing the pharmaceutical formulation is simple and easy to perform. To obtain said formulation, it is necessary to subject the calcium stearate to micronization before preparing the formulation.

The steps for preparing the formulation according to the invention are as follows:
- micronizing glyceryl monostearate or calcium stearate to a particle size between 30 and 60 µm;
- dissolving the preservatives in the mineral oil while stirring;
- slowly adding glyceryl monostearate or micronized calcium stearate while stirring at 50 to 200 rpm, and then adding anatase titanium dioxide slowly while still stirring;
- maintaining constant stirring between 80 and 120 rpm for 1 to 3 h.
- unloading the product and filling it in 4g syringes.

According to a preferred embodiment of the invention, the preparation method comprises the following steps:
- micronizing glyceryl monostearate, or salt derived from stearic acid, or calcium stearate, to a particle size between 30 and 100 µm;
- dissolving 0.1 to 0.6% by weight of preservatives in 30 to 70% by weight of mineral oil, under stirring;
- slowly adding, while stirring, 20 to 70% by weight of micronized calcium stearate; and then slowly adding 2 to 10% by weight of anatase titanium dioxide under stirring;
- maintaining constant stirring between 80 and 120 rpm for 1 to 3 h.
- unloading the product and filling it into 4 g syringes.

Even more preferably, the method of preparing the intramammary sealing composition comprises the following steps:
- micronizing glyceryl monostearate, or calcium stearate, to a particle size between 30 and 60 µm;
- dissolving 0.05 to 0.3% by weight of butylhydroxytoluene or butylhydroxyanisole in 40 to 60% by weight of mineral oil, under stirring;
- adding slowly and with stirring, 40 to 50% by weight of micronized calcium stearate; and then slowly adding 2 to 4% by weight of anatase titanium dioxide under stirring;
- maintaining constant stirring between 80 and 120 rpm for 1 to 3 h.
- unloading the product and filling it in 4 g syringes.

The following examples serve to clarify the scope of the invention and should not be taken for limiting purposes of the invention.

### EXAMPLE 1

The formulations of the present invention were obtained as described in the following steps: in a stand mixer, mineral oil (33.2g) was added, under stirring at 100 RPM, BHT (0.12g) and BHA (0.12g). Agitation was maintained at 100 rpm for 10 minutes. Then, micronized calcium stearate (46g) was added, with a particle size of 44.5 µm. Then add Anatase titanium dioxide (1.8g). It was stirred at 100 rpm for 2h. The product obtained was unloaded and filled into 4g syringes.

### EXAMPLE 2

In a stand mixer add mineral oil (30g), stirring at 100 rpm, BHT (0.20g) and BHA (0.20g). Agitation was maintained at 100 rpm for 10 minutes. Then add micronized calcium stearate (40g), with a particle size of 44.5 µm. Then, Anatase titanium dioxide (2.1g) was added and stirred at 100 rpm for 2h. The product obtained was unloaded and filled into 4g syringes.

A pharmaceutical formulation is considered stable if it passes stability tests under controlled temperature conditions or under stress.

The stability test of the sealing composition obtained was carried out, using a temperature of 50°C ± 2°C for 7 days, as well as 10°C ± 2°C for 7 days and the results demonstrated that the formulation remained stable within the limits required for approval.

## Claims

1. Intramammary teat sealant composition, is **characterized** because said composition is injectable and comprises organic salt of glyceryl monostearate or calcium stearate, with particle size between 30 and 100µm, in addition to mineral oil, titanium dioxide and stabilizer, in the absence of water.

2. Intramammary teat sealing composition according to claim 1, is **characterized by** using organic salt of glyceryl monostearate or calcium stearate, with particle size between 30 and 60µm.

3. Injectable intramammary teat sealing composition according to claim 1, is **characterized by** employing anatase titanium dioxide.

4. Intramammary teat sealing composition according to claim 1, is **characterized** as said organic salt comprises, by comprising mass, between 20 and 70% of the total mass of the sealing composition.

5. Intramammary teat sealing composition according to claim 1, is **characterized** because the sealing composition comprises in mass in relation to the total mass of the composition:
- mineral oil between 30 and 70.0% (m/m);
- calcium stearate between 20 and 50.0% (m/m);
- titanium dioxide between 2.0 to 10.0% (m/m);
- preservatives between 0.05 to 0.60% (m/m).

6. Intramammary teat sealing composition according to claim 1, is **characterized** because the preservatives can be anisoles or parabens.

7. Intramammary teat sealing composition, according to any of the above, is **characterized** because the sealing composition may comprise:
- mineral oil between 40 and 60.0% (m/m);
- calcium stearate between 40 and 50.0% (m/m);
- titanium dioxide between 2.0 to 4.0% (m/m);
- butylhydroxytoluene 0.05 to 0.30% (m/m);
- butylhydroxyanisole 0.05 to 0.30% (m/m).

8. Intramammary teat sealing composition according to claim 1 is **characterized** because said intramammary sealing composition comprises:
- mineral oil between 40 and 60.0% (m/m);
- calcium stearate between 40 and 50.0% (m/m);
- titanium dioxide between 2.0 to 4.0% (m/m);
- butylhydroxytoluene 0.05 to 0.30% (m/m);
- butylhydroxyanisole 0.05 to 0.30% (m/m).

9. Method of preparing intramammary teat sealing composition, is **characterized by** comprising the following steps:
- micronizing glyceryl monostearate or salt derived from stearic acid, calcium, to a particle size between 30 and 100 µm (component a);
- dissolving the preservatives in the mineral oil while stirring;
- slowly adding, while stirring, the micronized calcium stearate (or component a); and then slowly adding anatase titanium dioxide under stirring;
- maintaining constant stirring between 80 and 120 rpm for 1 to 3 h.
- unloading the product and filling it into syringes.

10. Method of preparing intramammary teat sealing composition, is **characterized by** comprising the following steps:
- micronizing glyceryl monostearate or calcium stearate to a particle size between 30 and 60 µm;
- dissolving the preservatives in the mineral oil while stirring;
- slowly adding glyceryl monostearate or micronized calcium stearate while stirring at 50 to 200 rpm, and then adding anatase titanium dioxide slowly while still stirring;
- maintaining constant stirring between 80 and 120 rpm for 1 to 3 h.
- unloading the product and filling it in 4g syringes.

11. Method of preparing an intramammary teat sealing composition according to claim 1, is **characterized by** comprising the following steps:
- micronizing glyceryl monostearate, or salt derived from stearic acid, or calcium stearate, to a particle size between 30 and 100 µm;
- dissolving 0.1 to 0.6% by weight of preservatives in 30 to 70% by weight of mineral oil, under stirring;
- slowly adding, while stirring, 20 to 70% by weight of micronized calcium stearate; and then slowly adding 2 to 10% by weight of anatase titanium dioxide under stirring;
- maintaining constant stirring between 80 and 120 rpm for 1 to 3 h.
- unloading the product and filling it in 4g syringes.

12. Method of preparing an intramammary teat sealing composition according to claim 1, is **characterized by** comprising the following steps:
- micronizing glyceryl monostearate, or calcium stearate, to a particle size between 30 and 60 µm;
- dissolving 0.05 to 0.3% by weight of butylhydroxytoluene or butylhydroxyanisole in 40 to 60% by weight of mineral oil, under stirring;
- slowly adding, while stirring, 40 to 50% by weight of micronized calcium stearate; and then slowly adding 2 to 4% by weight of anatase titanium dioxide under stirring;
- maintaining constant stirring between 80 and 120 rpm for 1 to 3 h.
- unloading the product and filling it in 4 g syringes.
